# EUROPEAN PATENT SPECIFICATION

(11) **EP 4 384 205 B1**
(45) Date of publication and mention of the grant of the patent: **14.01.2026**
(21) Application number: 22764390.5
(22) Date of filing: 09.08.2022
(51) Int. Cl.: A61K 38/37, A61K 9/00, A61K 9/19, A61K 47/18, A61P 7/04, A61K 9/08, A61K 9/16

(54) **METHOD FOR PRODUCING HUMAN PLASMA-DERIVED FACTOR VIII / VON WILLEBRAND FACTOR AND COMPOSITION OBTAINED**
VERFAHREN ZUR HERSTELLUNG DES AUS MENSCHLICHEM PLASMA ABGELEITETEN FAKTORS VIII/VON-WILLEBRAND-FAKTORS UND ERHALTENE ZUSAMMENSETZUNG
PROCÉDÉ DE PRODUCTION DE FACTEUR VIII / FACTEUR DE VON WILLEBRAND DÉRIVÉ DU PLASMA HUMAIN ET COMPOSITION OBTENUE

(30) Priority: 11.08.2021 EP 21382756
(43) Date of publication of application: 19.06.2024
(73) Proprietor: Grifols Worldwide Operations Limited, Clondalkin, Dublin 22 (IE)
(72) Inventor: GRANCHA GAMON, Salvador, 08150 PARETS DEL VALLES (BARCELONA) (ES); FARO TOMAS, Maria Mercedes, 08150 PARETS DEL VALLES (BARCELONA) (ES); MARTINEZ CREUS, Nuria, 08150 PARETS DEL VALLES (BARCELONA) (ES)
(74) Representative: ABG Intellectual Property Law, S.L.
(86) International application number: PCT/EP2022/072325
(87) International publication number: WO 2023/017020

(56) References cited:
- EP-B1- 1 522 312
- WO-A1-00/48635
- WO-A1-93/22336

## Description

The field of the present invention relates to a new method and pharmaceutical composition obtained with said method comprising human plasma-derived coagulation Factor VIII / von Willebrand Factor (FVIII/VWF) complex at higher potency (IU/ml) and higher doses (IU/vial) per vial of both active ingredients FVIII and VWF, as compared with those currently available in the market. Moreover, the new formulation step allows solubilizing the FVIII/VWF complex, lyophilizing, and reconstituting prior to administration at higher FVIII and VWF potency resulting in less injection volume for the treatment of Hemophilia A and for the treatment of Von Willebrand Disease.

FVIII concentrates are currently available in the market containing both FVIII and VWF or as FVIII without or with low content of VWF. Only the first group, which includes products derived from pools of human plasma in which FVIII and VWF are tightly bound forming a natural complex (FVIII/VWF), are indicated for either Hemophilia A and Von Willebrand Disease treatment.

In the blood stream, the presence of VWF plays a crucial dual role on preserving FVIII functionality through the protection from degradation by plasma proteinases as well as by interfering with antigen recognition by FVIII inhibitors (Delignat S. et al. Immunoprotective effect of von Willebrand Factor towards therapeutic factor VIII in experimental Haemophilia A. Haemophilia 2012; 18: 248-254). When assessing the immunogenicity of FVIII products, not only the origin of the FVIII (recombinant or plasma-derived), but also the presence of VWF in the form of the physiologically formed FVIII/VWF complex, seems to be important in the FVIII recognition by inhibitors [Peyvandi F, et al. Source of Factor VIII Replacement (plasmatic or recombinant) and Incidence of Inhibitory Alloantibodies in Previously Untreated Patients with Severe Hemophilia A: The Multicenter Randomized Sippet Study. Blood 2015; 126: 5, and Bravo et al. Neutralizing capacity of inhibitors on FVIII is lower for natural FVIII/VWF complex than for isolated FVIII: in vitro comparability study in eleven different therapeutic FVIII concentrates. Haemophilia 2016; 22: e301-e348].

On the other hand, VWF constitute the largest known soluble plasma protein. It has a complex multimeric structure, with a molecular weight that varies between 460 kDa in dimeric form, which is the smaller plasmatic form, up to more than 20,000 kDa for multimeric forms of greater molecular weight.

As mentioned above, there are several products containing FVIII/VWF available in the market, which are usually presented as a lyophilized powder for solution for injection containing nominally 250, 500, 1000 or 1500 IU human coagulation factor VIII per vial. These products are administrated to the patient by an intravenous route and the volume to be infused is directly depending on the available dose and the potency of the FVIII or VWF once reconstituted. In practice, several milliliters have to be infused to a patient in a normal treatment by slow intravenous (IV) injection. Therefore, there is still the need to reduce the volume to be administered to the patient, reducing the duration of the treatment with the associated therapeutic benefit.

In this context, attempts to increase potency of FVIII concentrated without altering the composition of the formula have been made, for example, by reducing the reconstitution volume of the lyophilized product. These attempts have been unsuccessful mainly due to the low solubility of VWF, which causes appearance of flocculates or increases the reconstitution time up to unacceptable values. Further attempts to increase solubility, by increasing the excipient concentrations, result in an unacceptable osmolality of the final product to be infused to the patient.

The inventors of the present invention have developed a new method for producing a pharmaceutical composition comprising human plasma-derived FVIII/VWF complex wherein in the formulation step the concentration of albumin has been slightly increased, while decreasing the concentration of histidine and arginine as compared with similar products already in the market, with which it is possible to increase potency of FVIII, and thus increasing the amount of FVIII, and thereby of VWF, up to 3,600 IU per vial, without affecting quality parameters of the final product, in particular osmolality and solubility. The inventors are not aware that such a presentation has been obtained before for any natural plasma-derived FVIII/VWF product. In addition, with the product obtained by the method of the present invention, it is possible to reduce the administration volume for all presentations, especially relevant in pediatric used doses (in which said volume reduction is up to 4 times as compared with similar products in the market), while, on the other hand, maintaining product osmolality within the tonicity range of current products, even for the high 3000 IU of FVIII per vial presentation. The osmolality increases with the dose and the potency of the product and therefore is especially relevant on higher therapeutic doses.

### SUMMARY

The present invention refers to a method for producing a lyophilized pharmaceutical composition comprising human plasma-derived Factor VIII/Von Willebrand Factor (FVIIIIVWF) complex, comprising the steps of:
a) providing an initial aqueous solution comprising FVIII/VWF with at least 90 IU of FVIII per mg of total proteins, with a ratio of VWF/FVIII activity of at least 0.7;
b) adding arginine, histidine, and human serum albumin (HSA);
c) lyophilizing the FVIII/VWF solution obtained in step b); and
d) reconstituting the lyophilized FVIII/VWF in water for injection thereby obtaining a therapeutic product adequate for the treatment of Hemophilia A and Von Willebrand Disease,
wherein prior to administration to a patient arginine is at a concentration between 42 and 98 mmol/L, histidine is at a concentration between 10 and 24 mmol/L, albumin is at a concentration between 1.0 and 2.5 % (w/v), FVIII potency and VWF potency are equal to or higher than 200 IU/mL, and the obtained product has a FVIII dose up to 3600 IU/vial and a VWF dose up to 5040 IU/vial.

Preferably, prior to administration, FVIII dose is between 2400 IU/vial and 3600 IU/vial and VWF dose is between 2160 IU/vial and 5040 IU/vial.

Preferably, albumin concentration in step d) is between 1.2 and 2.3 % (w/v).

Preferably, histidine concentration in step d) is between 13 and 21 mmol/L.

Preferably, arginine concentration in step d) is between 50 and 90 mmol/L.

Preferably, the method of the present invention further comprises a step of heat treatment. Said heat treatment can be performed for example at a temperature from 70 ºC to 90 ºC, more preferably between 75 ºC and 85 ºC, even more preferably between 80 ºC and 82 ºC, for 70-80 h, more preferably between 72-74 h. More preferably, the FVIII/VWF complex is not affected by said heat treatment and the heated product is similar to the FVIII/VWF complex present in plasma.

Preferably, osmolality of the product obtained in step d) is equal to or higher than 240 mOsm/kg. More preferably, said osmolality is between 240 and 600 mOsm/kg, even more preferably between 240 and 500 mOsm/kg, most preferable between 240 and 400 mOsm/kg even for 3000 IU dose. Preferably, reconstitution time in step d) is less than 10 min. More preferably, reconstitution time in step d) is less than 5 min, even more oreferably less than 3 min.

In another aspect, the present invention discloses a composition comprising lyophilized FVIII/VWF complex wherein concentration of arginine after reconstitution is between 42 and 98 mmol/L, concentration of histidine after reconstitution is between 10 and 24 mmol/L, and concentration of human serum albumin (HSA) after reconstitution is between 1.0 and 2.5 % (w/v), and wherein the FVIII potency and VWF potency are equal to or higher than 200 IU/mL, and a FVIII dose up to 3600 IU/vial and a VWF dose up to 5040 IU/vial.

Preferably in said composition, albumin concentration is between 1.2 and 2.3 % (w/v). Preferably, histidine concentration is between 13 and 21 mmol/L. Preferably, arginine concentration is between 50 and 90 mmol/L.

Preferably in said composition, FVIII dose is between 2400 IU/vial and 3600 IU/vial and VWF dose is between 2160 IU/vial and 5040 IU/vial.

Preferably, osmolality of composition is equal to or higher than 240 mOms/kg. More preferably, said osmolality is between 240 and 600 mOsm/kg, even more preferably between 240 and 500 mOsm/kg, most preferable between 240 and 400 mOsm/kg. Preferably, reconstitution time of the lyophilized composition is less than 10 min. More preferably, reconstitution time in step d) is less than 5 min, even more preferably less than 3 min.

Preferably, the composition of the present invention has been heat treated. Said heat treatment can be performed for example at a temperature from 70 ºC to 90 ºC, more preferably between 75 ºC and 85 ºC, even more preferably between 80 ºC and 82 ºC, for 70-80 h, more preferably between 72-74 h. More preferably, the FVIII/VWF complex is not affected by said heat treatment and is similar to the FVIII/VWF complex present in plasma.

Preferably, the composition of the present invention has a stability at 5 ºC or 30 ºC of at least 36 months, and at 40 ºC of at least 12 months.

In a further aspect, the present invention refers to a composition as described above for use in the treatment of Hemophilia A and von Willebrand Disease.

### DETAILED DESCRIPTION

### Definitions

As used herein, the section headings are for organizational purposes only and are not to be construed as limiting the described subject matter in any way. All literature and similar materials cited in this application, including but not limited to, patents, patent applications, articles, books, treatises, and internet web pages are expressly incorporated by reference in their entirety for any purpose. When definitions of terms in incorporated references appear to differ from the definitions provided in the present teachings, the definition provided in the present teachings shall control. It will be appreciated that there is an implied "about" prior to the temperatures, concentrations, times, etc. discussed in the present teachings, such that slight and insubstantial deviations are within the scope of the present teachings herein.

In this application, the use of the singular includes the plural unless specifically stated otherwise. Also, the use of "comprise", "comprises", "comprising", "contain", "contains", "containing", "include", "includes", and "including" are not intended to be limiting.

As used in this specification and claims, the singular forms "a," "an" and "the" include plural references unless the content clearly dictates otherwise.

The term "treatment" or "treating" means any treatment of a disease or disorder in a subject, such as a mammal, including: preventing or protecting against the disease or disorder, that is, causing the clinical symptoms not to develop; inhibiting the disease or disorder, that is, arresting or suppressing the development of clinical symptoms; and/or relieving the disease or disorder that is, causing the regression of clinical symptoms.

Although this disclosure is in the context of certain embodiments and examples, those skilled in the art will understand that the present disclosure extends beyond the specifically disclosed embodiments to other alternative embodiments and/or uses of the embodiments and obvious modifications and equivalents thereof. In addition, while several variations of the embodiments have been shown and described in detail, other modifications, which are within the scope of this disclosure, will be readily apparent to those of skill in the art based upon this disclosure.

It is also contemplated that various combinations or sub-combinations of the specific features and aspects of the embodiments may be made and still fall within the scope of the disclosure. It should be understood that various features and aspects of the disclosed embodiments can be combined with, or substituted for, one another in order to form varying modes or embodiments of the disclosure. Thus, it is intended that the scope of the present disclosure herein disclosed should not be limited by the particular disclosed embodiments described above.

It should be understood, however, that this description, while indicating preferred embodiments of the disclosure, is given by way of illustration only, since various changes and modifications within the spirit and scope of the disclosure will become apparent to those skilled in the art.

The terminology used in the description presented herein is not intended to be interpreted in any limited or restrictive manner. Rather, the terminology is simply being utilized in conjunction with a detailed description of embodiments of the systems, methods and related components. Furthermore, embodiments may comprise several novel features, no single one of which is solely responsible for its desirable attributes or is believed to be essential to practicing the embodiments herein described.

The term "FVIII potency" in the present disclosure refers to factor VIII:C potency (IU/mL) as determined using the European Pharmacopoeia chromogenic assay, which is well-known to the skilled person. The term "human plasma-derived" in the present disclosure refers to the source of the starting material to obtain the product, i.e. human plasma, using any conventional or non-conventional human plasma fractionation process. One example of a conventional plasma fractionation process is the Cohn's method.

The term "VWF potency" in the present disclosure refers to von Willebrand Factor potency (IU/mL) as determined using the ristocetin cofactor assay as described in the European Pharmacopoeia, which is well-known to the skilled person.

### Process for obtaining human plasma-derived FVIII/VWF complex

The process for obtaining human plasma-derived factor VIII is very similar to the process disclosed in US 8354505 B2, until the step of preparing the high purity FVIII/VWF complex in which different excipients concentrations are adjusted. Firstly, the human plasma is pooled and cryoprecipitated using plasma from more than 1,000 donors, and then is further purified downstream to a high purity FVIII precipitate. Said high purity precipitate is resuspended in an albumin, arginine and histidine solution. After resuspension, the albumin, histidine and arginine concentration as well as the pH is readjusted to the established value in the sterile bulk. FVIII activity is finally adjusted to the sterile bulk potency predetermined nominal value and the VWF activity is thereby adjusted. Said solution is filtered through a gradient of filters down to 0.22 µm sterile filter membranes to obtain the sterile bulk solution. Said solution is then filled into vials according to the desired presentation. In the present invention it is possible to fill up to 3,000 IU of FVIII per vial, which is not available on similar products in the market, which are then lyophilized and heat treated to obtain the final FVIII/VWF product. For the intravenous application, said lyophilized product is reconstituted in water for injection (WFI) to the desired potency. With the composition of the present invention, the reconstitution volume after lyophilization can be reduced between half and four times as compared to similar products in the market.

### Effect of albumin in FVIII/VWF stabilization

It is known that albumin would improve solubility and stabilization of FVIII/VWF during the purification process as well as during the shelf life of the product. On the other hand, osmolality is critical issue in the pharmaceutical products which must be characterized. Surprisingly, small increments of albumin concentration in the sterile bulk prior lyophilization, causing minimal impact on the osmolality of the final product, substantially improve the solubility of the final product before administration. The increase of albumin concentration, which is not too high to cause undesirable side effects, surprisingly allows reducing the concentration in the amino acids histidine and arginine in the composition of the present invention reducing the overall tonicity of the product prior to administration.

Having now generally described the invention, the same will be more readily understood through reference to the following examples, which are provided by way of illustration, and are not intended to be limiting of the present invention.

### EXAMPLES

Example 1. Process of obtention of the natural FVIII/VWF complex subjected to the formulation of the invention:
The natural FVIII/VWF complex solution, obtained from the cryoprecipitate generated when thawing human plasma, can be purified, for example, from the solubilized cryoprecipitate suspension after being purified by polyethylene glycol (PEG) precipitation and subsequent purification by chromatography, as shown in Patent US 8354505 B2. The eluted solution is concentrated and subsequently further purified to obtain a high purity FVIII/VWF complex, for example by precipitation with Glycine, as shown in Patent EP 0639203.

The obtained High purity intermediate preserves the suitable characteristics to be formulated with excipients and stabilizers down to the bulk. The FVIII/FVW bulk solution is filled into vials according to the desired dose, freeze-dried and subject to a final Heat treatment in order to obtain a virally free and long term stable final product (3 years at RT), defined as natural *FVIII*/*VWF* complex.

**TABLE 1**

| PROCESS INTERMEDIATE | SPECIFIC ACTIVITY | | FVW:RCo/FVIII (IU/IU)⁽³⁾ |
|---|---|---|---|
| | IU FVIII/AU₂₈₀ ⁽¹⁾ | IU FVIII/mg protein ⁽²⁾ | |
| CRYOPRECIPITATE SUSPENSION | 0.55±0.09 | - - | 1.2±0.2 |
| PARTIALLY PURIFIED INTERMEDIATE | 14.3±2.1 | - - | - - |
| HIGH PURITY INTERMEDIATE PRIOR TO FORMULATION | 76.2±5.3 | 105.4±4.9 | 1.3±0.1 |

| | | | |
|---|---|---|---|
| **(1) Specific** activity is determined using the FVIII units divided by the approximate protein determined by optical density at 280 nm (2) For the High Purity Intermediate, specific activity is also determined as the FVIII units divided by the total protein. The total protein is determined assuming an extinction coefficient of ε_{1 %} 13.64 cm-1 (3) The FVW activity is determined as Ristocetin Cofactor activity of Von Willebrand factor (FVW:RCo). | | | |

In conclusion, the functionality activity ratio FVW:RCo/FVIII preserved along the process (table 1) together with the purity eventually obtained, lead to a High purity *FVIII*/*VWF* intermediate suitable to be formulated with the formulation of the invention thereby obtaining a stable natural *FVIII*/*VWF* complex, resembling as it is in plasma, with its corresponding therapeutic indications.

The above detailed process for obtaining the high purity intermediate was used in all the formulation examples of the present invention. VWF activity was measured by using Ristocetin cofactor activity, giving a ratio of 1.3±0.1 at all cases. Therefore, at all examples, 1 IU of FVIII involve approximately 1.3 IU of VWF activity.

Example 2. Impact on FVIII/VWF solubility when increasing both factors; FVIII potency (IU/ml) and dose (IU/vial) with a currently available state of the art composition (100 mM Arg, 25 mM Hist, 0.5 % Alb).

The High purity FVIII/VWF obtained as described in example 1 was formulated in bulk and freeze-dried so that the final product, prior to administration is an aqueous formulation equivalent to prior art formula i.e. 100 mM Arg, 25 mM His and 0.5 % of Albumin.

In order to assess the impact of both factors, dose (UI/vial) and administration potency (UI/vial) on FVIII/VWF solubility, the formulated bulk was filled in the 3000 UI dose and reconstituted at both potencies 100 and 200 UI/ml. Moreover, as a control, part of the bulk was filled in vials within the smaller doses existing in prior art (1500 and 2000 IU) and solubility parameters were evaluated. The final freeze-dryed products were solubilized at either 100 UI/ml or 200 UI/ml displaying very different solubilizing parameters:
A) Prior art doses and potency
B) 3000 IU /vial reconstituted at 100 IU/ml of FVIII
C) 3000 IU/vial reconstituted at 200 IU/ml of FVIII

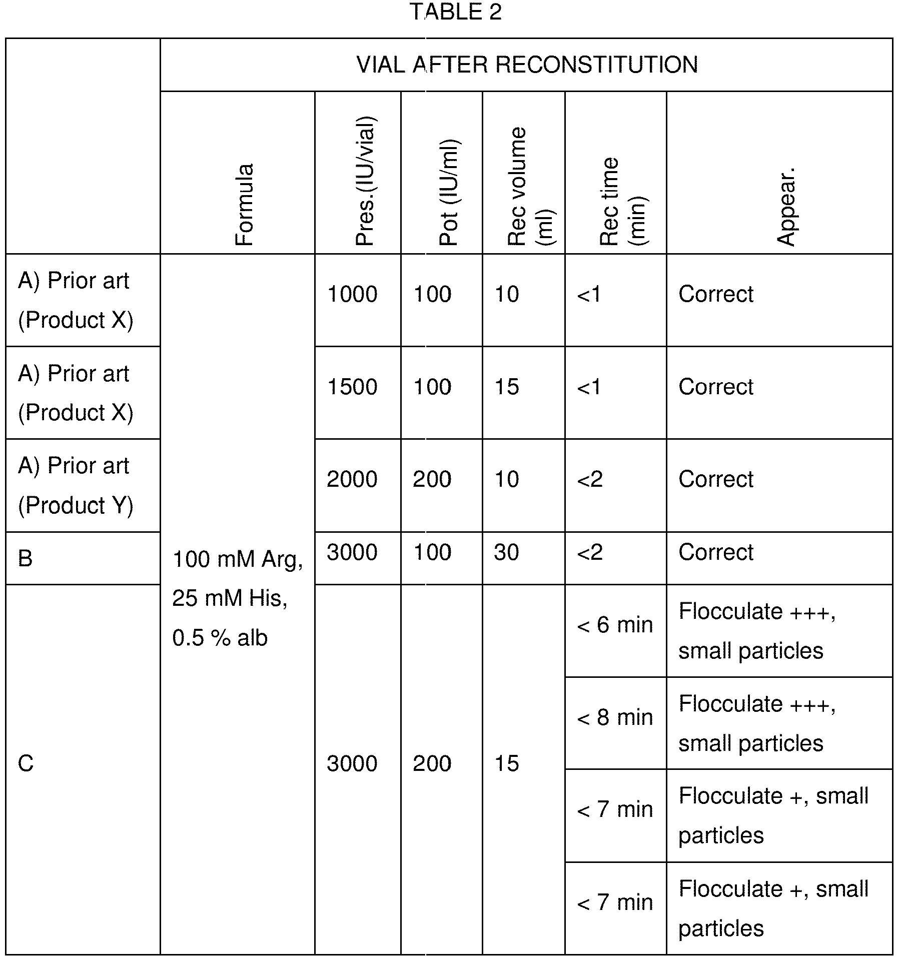

In conclusion, the prior art composition, 100 mM Arg, 25 mM His, 0.5 % albumin, shown to be adequate to solubilize the FVIII/FVW at 100 IU FVIII/mL even for a large presentation of 3000 IU, but it is not able to solubilize the FVIII/FVW complex at 200 IU FVIII /ml for the same dose of 3000 IU (i.e. larger reconstitution times and appearance with flocculates are observed). Thus, both activity factors, the total dose (UI/vial) and the potency per ml (UI/ml) do contribute to difficult the solubility of the product prior to administration.

Example 3. Solubility and Osmolality balance on the FVIII/VWF natural complex: Impact of increasing twice the concentration of albumin, arginine and histidine.

In order to assess the impact on increasing only albumin and increasing both albumin and amino acids on solubility for 3000 IU, the High purity FVIII/VWF intermediate obtained as described in the example 1 was formulated in order to obtain in each case a freeze-dried product wherein said formulation following reconstitution is an aqueous formulation equivalent to:
- FORMULA A) 100 mM Arg, 25 mM His and 0.5 % alb reconstituted at 200 IU FVIII /ml
- FORMULA B) 100 mM Arg, 25 mM His and 1 % alb reconstituted at 200, 250 and 300 IU FVIII /ml.
- FORMULA C) 200 mM Arg, 50 mM His, and 1 % alb reconstituted at 200, 250 and 300 IU FVIII/ml.

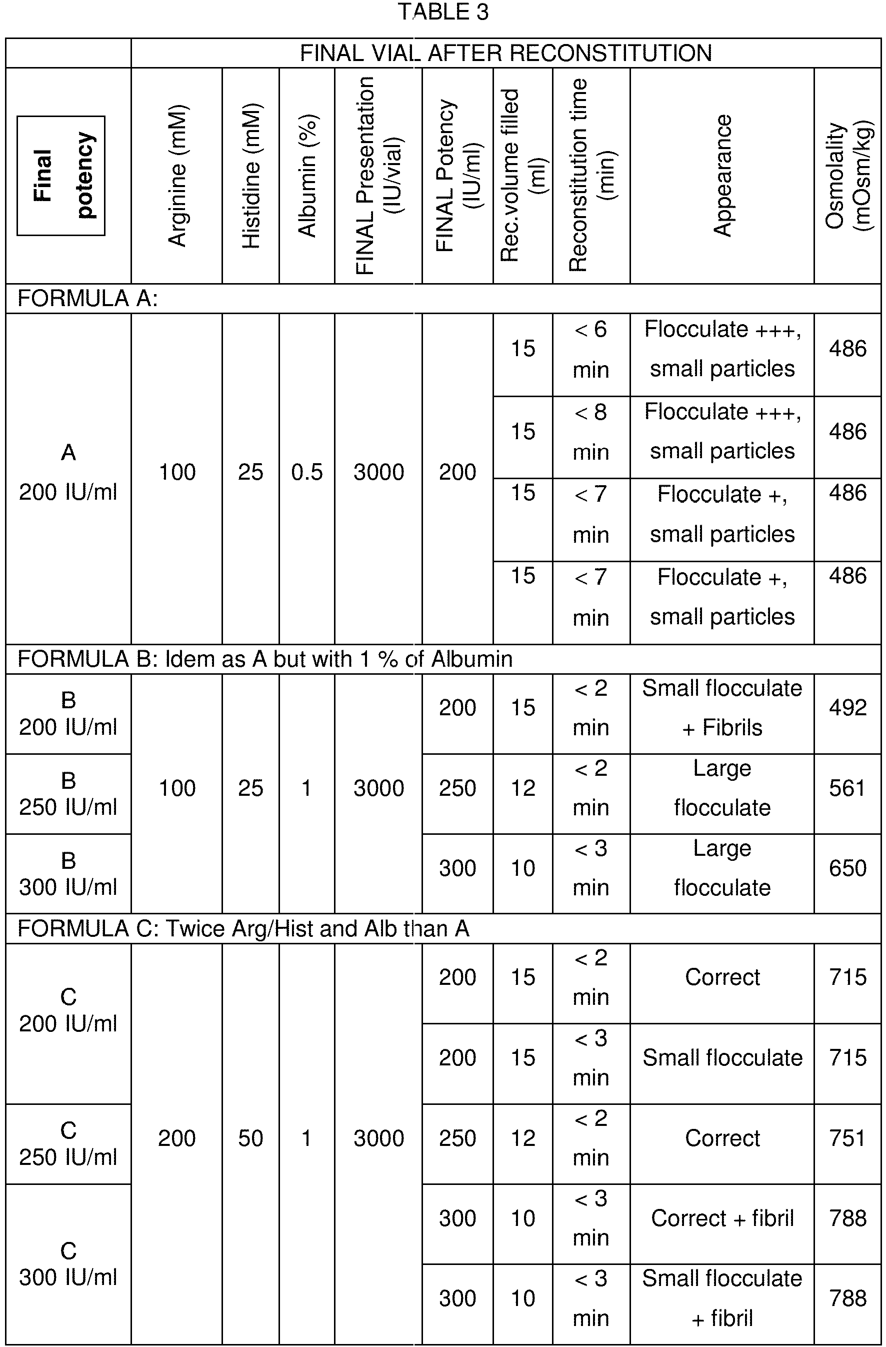

Noticeably, the increase of albumin leads to a remarkable improvement on solubility with negligible impact on osmolality (Formulation B). However, the additional increase of excipients (Arginine up to 200 mM and histidine up to 50 mM) while maintaining this 1 % albumin, unexpectedly do not provide any additional improvement on solubility (Formulation C *vs.* B) while the impact on final product osmolality values is very high (Formula C vs B).

In conclusion, it has been assessed the unexpected benefit in solubility by only increasing albumin while maintaining other amino acids in the present formula, with the additional advantage on preserving osmolality.

Example 4. Solubility and Osmolality balance on the FVIII/VWF natural complex: Impact of increasing Albumin up to 2 % and increasing amino acids 1.5-fold

High purity FVIII/VWF intermediates obtained as described in the example 1 were formulated in order to obtain in each case a freeze-dried product which after reconstitution is equivalent to the following aqueous formulation:
A) 100 mM Arg, 25 mM His and 0.5 % alb was reproduced as control (prior art formula)
B) 150 mM Arg, 37.5 mM His, and 2 % alb: Increase of albumin and aminoacids 1.5 fold

For the two formulations, the dose of 3000 IU/vial at high potency (200 IU FVIII/ml) was tested:

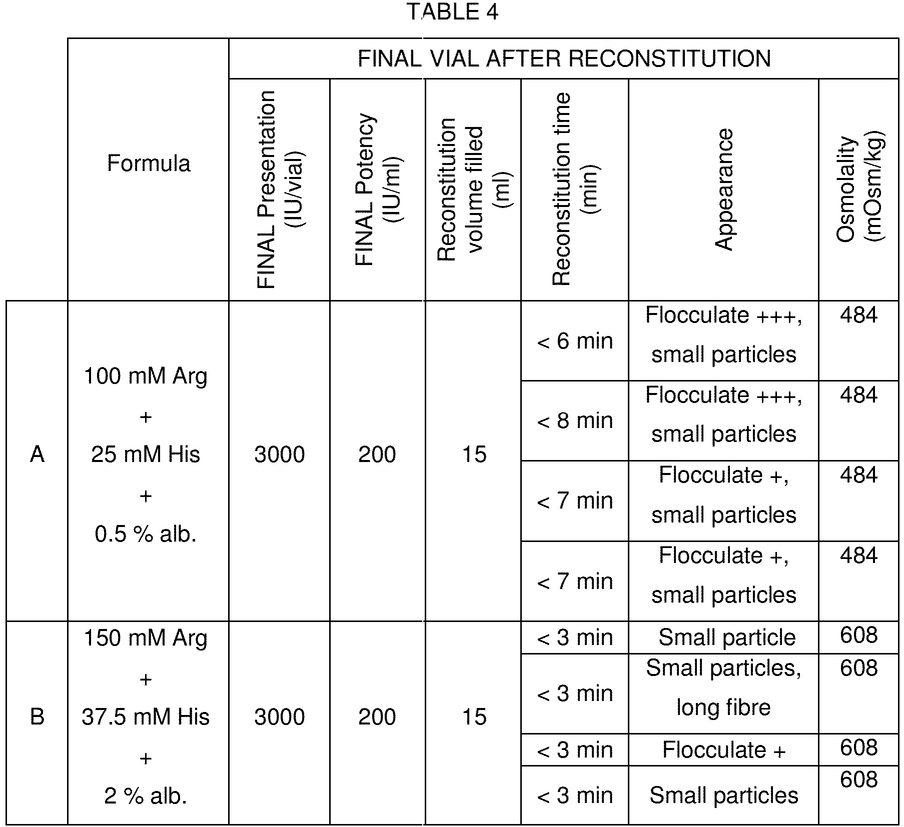

The results obtained shows that the increase in albumin up to 2 % and aminoacids (Arginine up to 150 mM and Histidine 37.5 mM) leads to a correct 3000 IU dose at high potency (200 IU FVIII/ml) in terms of solubility (reconstitution time and appearance of the reconstituted solution). However, the evaluated composition (150 mM Arg, 37 mM Hist, 2 % albumin) lead also to an osmolality increase that was not desired.

Example 5. Solubility and Osmolality balance on the FVIII/VWF natural complex. Impact of Albumin/Arginine ratio on osmolality while maintaining solubility at 3000 IU dose and 200 UI/ml potency.

High purity FVIII/VWF intermediates obtained as described in the example 1 were formulated in order to obtain a freeze-dried product which after reconstitution is equivalent to the following aqueous formulations:
- A) 200 mM Arg, 50 mM His and 1 % alb (Ratio Alb/Arg: 0.05)
- B) 110 mM Arg, 22 mM His, and 1.8 % alb (Ratio Alb/Arg:0.16)
- C) 70 mM Arg, 17 mM His, and 1.8 % alb (Ratio Alb/Arg: 0.26)

These formulations were tested at 3000 IU/vial dose at high FVIII and FVW potency (nominal 200 IU/ml of FVIII):

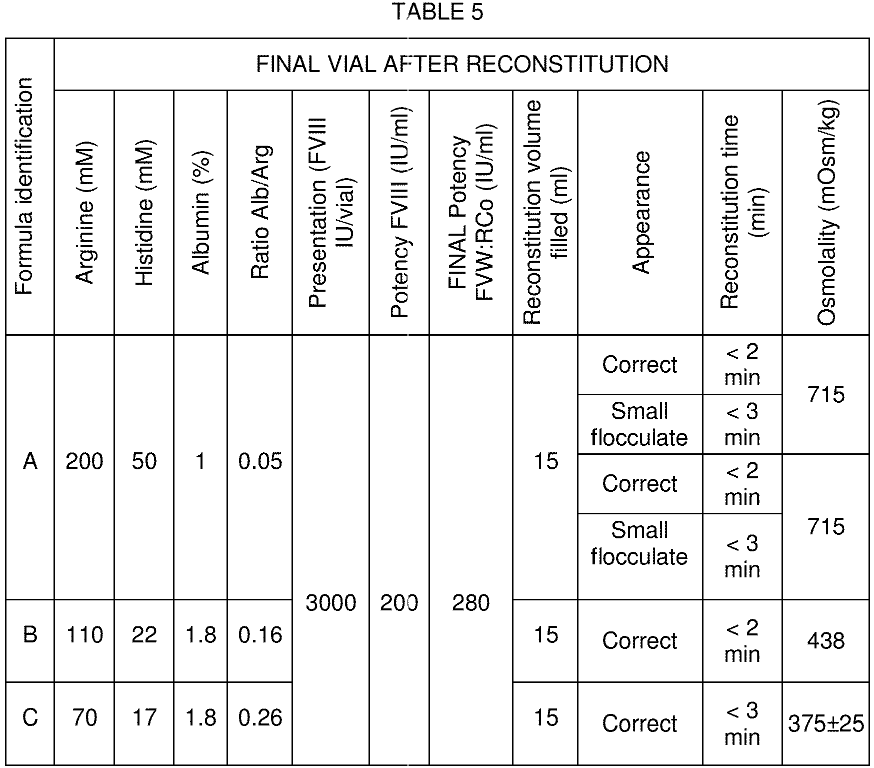

Surprisingly, the increase in albumin up to 1.8 % allows the reduction of the concentration of amino acids in the formulation (70 mM Arg and 17 mM His) while maintaining the solubility criteria and providing a minimum impact on osmolality for a very high dose of 3000 IU and at high FVIII potency.

In conclusion, the high purity intermediate of FVIII/VWF obtained as example 1, formulated with Arginine 70 mM, Hist 17 mM and albumin 1.8 %, and with a perfectly balanced Alb/Arg ratio is able to eventually lead a product with a dose of 3000 UI/ml of FVIII, 4200 UI/ml of FVW, that can be administered with 15 ml and with a final osmolality less than 400 mOsm/kg and with a reconstitution time lower than 3 min.

Example 6. Suitability of the formulation of the present invention to be subjected a heat treatment on the freeze-dried product preserving both FVIII and FVW activity.

Different High purity FVIII/VWF intermediates from lots of 6800 L of plasma at industrial scale were obtained as described in the example 1. The HP FVIII/FVW complex intermediates, were formulated with the formula of the invention and were filled into 250, 500, 1000, 2000 and 3000 IU vials. Vials were subjected to freeze-drying in order to ensure a correct moisture, and subsequently to a heat treatment with the capacity to inactivate viruses at 81±1 °C during 72-74 hours. All the doses were reconstituted at a nominal potency of 200 IU/ml, except the 250 IU that was reconstituted at 100 IU/ml (final formula: 70 mM Arg, 17 mM His, 1.8 % alb).

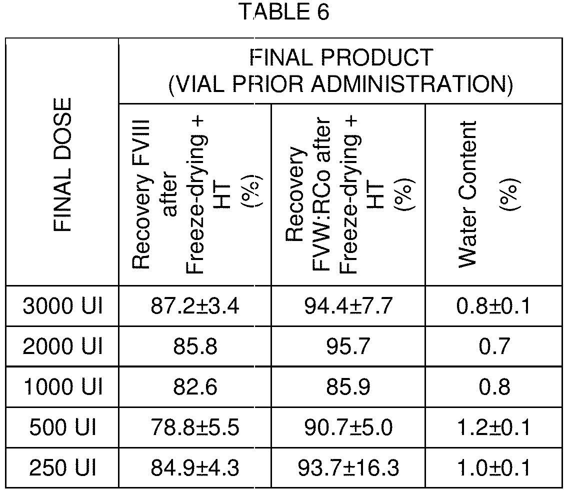

The results show that the formulatior of the invention is suitable to protect the FVIII/VWF complex during the Heat treatment, preserving the biological activity of both FVIII and FVW:RCo. Thus, the ratio of FVW:RCo/FVIII, indicative of the functional state of the complex, is thereby maintained after this heat treatment step resembling as it is in plasma, leading and stable, safe, soluble natural FVIII/VWF complex, with its corresponding therapeutic indications.

Example 7. Long term stability of the *FVIII*/*VWF* complex formulated with the present invention formula.

250, 500, 1000, 2000 and 3000 IU vials of FVIII/VWF were obtained. These final products were subjected to a long-term stability study at 5 ºC and 30 ºC for 36 months and 40 ºC for 12 months. Results are shown in Table 7.

**TABLE 7**

| | | **5 ºC** | | **30 ºC** | | **40 ºC** | |
|---|---|---|---|---|---|---|---|
| | **TEST** | Initial | 36M | Initial | 36M | Initial | 12M |
| **250 IU** | DISSOLUTION TIME (20-25ºC) | <2 | <1 | <2 | <1 | <2 | <2 |
| | FVIII ACTIVITY (%) | 100 | 98.7 | 100 | 87.4 | 100 | 90.9 |
| | RISTOCETIN COFACTOR (vWF:RCo) (%) | 100 | 97.6 | 100 | 100 | 100 | 90.4 |
| **500 IU** | DISSOLUTION TIME (20-25ºC) | <3 | <2 | <3 | <2 | <3 | <2 |
| | FVIII ACTIVITY (%) | 100 | 93.6 | 100 | 87.6 | 100 | 90.1 |
| | RISTOCETIN COFACTOR (vWF:RCo) (%) | 100 | 81.8 | 100 | 85.2 | 100 | 92.3 |
| **1000 IU** | DISSOLUTION TIME (20-25 ºC) | <3 | <2 | <3 | <2 | <3 | <3 |
| | FVIII ACTIVITY (%) | 100 | 100 | 100 | 100 | 100 | 100 |
| | RISTOCETIN COFACTOR (vWF:RCo) (%) | 100 | 87.2 | 100 | 87.9 | 100 | 100 |
| **2000 IU** | DISSOLUTION TIME (20-25% ºC) | <3 | <2 | <3 | <2 | <3 | <2 |
| | FVIII ACTIVITY (%) | 100 | 100 | 100 | 100 | 100 | 98.1 |
| | RISTOCETIN COFACTOR (vWF:RCo) (%) | 3162 | 3080 | 3162 | 2758 | 3162 | 3235 |
| **3000 IU** | DISSOLUTION TIME (20-25% ºC) | <3 | <2 | <3 | <2 | <3 | <2 |
| | FVIII ACTIVITY (%) | 100 | 96.6 | 100 | 90.2 | 100 | 90.0 |
| | RISTOCETIN COFACTOR (vWF:RCo) (%) | 100 | 96.8 | 100 | 100 | 100 | 100 |

The results of the functional state of the complex (FVIII and FVW:RCo activity) as well as the solubility parameters (reconstitution time) are shown in table 7 under the different temperature conditions and along time.

The results show that the formulatior of the invention is suitable to protect the FVIII/VWF complex along time under different temperature conditions. The biological activity of both FVIII and FVW is preserved at least up to 36 months at 5 and 30 ºC and up to 12 months at 40 ºC and the solubility is maintain unaltered along time.

## Claims

1. Method for producing a lyophilized pharmaceutical composition comprising human plasma-derived Factor VIII/Von Willebrand Factor (FVIII/VWF) complex, comprising the steps of:
a) providing an initial aqueous solution comprising *FVIII*/*VWF* with at least 90 IU of FVIII per mg of total proteins, with a ratio of VWF/FVIII activity of at least 0.7;
b) adding arginine, histidine, and human serum albumin (HSA);
c) lyophilizing the *FVIII*/*VWF* solution obtained in step b); and
d) reconstituting the lyophilized *FVIII*/*VWF* in water for injection thereby obtaining a therapeutic product adequate for the treatment of Hemophilia A and Von Willebrand Disease,
wherein prior to administration to a patient arginine is at a concentration between 42 and 98 mmol/L, histidine is at a concentration between 10 and 24 mmol/L, albumin is at a concentration between 1.0 and 2.5 % (w/v), FVIII potency and VWF potency are equal to or higher than 200 IU/mL, and the obtained product has a FVIII dose up to 3600 IU/vial, and a VWF dose up to 5040 IU/vial.

2. Method, according to claim 1, wherein albumin concentration in step d) is between 1.2 and 2.3 % (w/v).

3. Method, according to claim 1 or 2, wherein histidine concentration in step d) is between 13 and 21 mmol/L.

4. Method, according to any of the preceding claims, wherein arginine concentration in step d) is between 50 and 90 mmol/L.

5. Method, according to any of the preceding claims, further comprising a step of heat treatment.

6. Method, according to claim 5, wherein the *FVIII*/*VWF* complex is not affected by said heat treatment and is similar to the *FVIII*/*VWF* complex present in plasma.

7. Method, according to any of the preceding claims, wherein osmolality of the product obtained in step d) is equal to or higher than 240 mOsm/kg.

8. Method, according to any of the preceding claims, wherein reconstitution time in step d) is less than 10 min.

9. Composition comprising lyophilized *FVIII*/*VWF* complex wherein concentration of arginine after reconstitution is between 42 and 98 mmol/L, concentration of histidine after reconstitution is between 10 and 24 mmol/L, and concentration of human serum albumin (HAS) after reconstitution is between 1.0 and 2.5 % (w/v), and wherein the FVIII potency and VWF potency are equal to or higher than 200 IU/mL, and a FVIII dose up to 3600 IU/vial and a VWF dose up to 5040 IU/vial.

10. Composition, according to claim 9, wherein albumin concentration is between 1.2 and 2.3 % (w/v).

11. Composition, according to claim 9 or 10, wherein histidine concentration is between 13 and 21 mmol/L.

12. Composition, according to any of claims 9-11, wherein arginine concentration is between 50 and 90 mmol/L.

13. Composition, according to any of claims 9-12, wherein FVIII dose is between 2400 and 3600 IU/vial and VWF dose is between 2160 and 5040 I\U/vial.

14. Composition, according to any claims 9-14, wherein reconstitution time of the lyophilized composition is less than 10 min.

15. Composition, according to any of claims 9-14, for use in the treatment of Hemophilia A and Von Willebrand Disease in a subject in need thereof.

## Patentansprüche

1. Verfahren zur Herstellung einer lyophilisierten pharmazeutischen Zusammensetzung, umfassend aus menschlichem Plasma gewonnenem Faktor-VIII/Von-Willebrand-Faktor (FVIII/VWF)-Komplex, umfassend die Schritte:
a) Bereitstellen einer wässrigen Ausgangslösung, umfassend FVIII/VWF mit mindestens 90 I.E. FVIII pro mg Gesamtprotein, mit einem Verhältnis der VWF/FVIII-Aktivität von mindestens 0,7;
b) Zugabe von Arginin, Histidin und humanem Serumalbumin (HSA);
c) Lyophilisierung der in Schritt b) erhaltenen FVIII/VWF-Lösung; und
d) Rekonstitution des lyophilisierten FVIII/VWF in Wasser für Injektionszwecke, um ein für die Behandlung von Hämophilie A und Morbus Von-Willebrand geeignetes Therapeutikum zu erhalten,
wobei Arginin vor der Verabreichung an einen Patienten eine Konzentration zwischen 42 und 98 mmol/l aufweist, Histidin eine Konzentration zwischen 10 und 24 mmol/l aufweist, Albumin eine Konzentration zwischen 1,0 und 2,5 % (w/v) aufweist, die FVIII-Potenz und die VWF-Potenz gleich oder höher als 200 IE/ml sind und das erhaltene Produkt eine FVIII-Dosis von bis zu 3600 IE/Fläschchen und eine VWF-Dosis von bis zu 5040 I.E./Fläschchen aufweist.

2. Verfahren nach Anspruch 1, wobei die Albuminkonzentration in Schritt d) zwischen 1,2 und 2,3 % (w/v) liegt.

3. Verfahren nach Anspruch 1 oder 2, wobei die Histidinkonzentration in Schritt d) zwischen 13 und 21 mmol/l liegt.

4. Verfahren nach einem der vorhergehenden Ansprüche, wobei die Argininkonzentration in Schritt d) zwischen 50 und 90 mmol/l liegt.

5. Verfahren nach einem der vorhergehenden Ansprüche, das ferner einen Schritt der Wärmebehandlung umfasst.

6. Verfahren nach Anspruch 5, wobei der FVIII/VWF-Komplex durch die Wärmebehandlung nicht beeinflusst wird und dem im Plasma vorhandenen FVIII/VWF-Komplex ähnlich ist.

7. Verfahren nach einem der vorhergehenden Ansprüche, wobei die Osmolalität des in Schritt d) erhaltenen Produkts gleich oder größer als 240 mOsm/kg ist.

8. Verfahren nach einem der vorhergehenden Ansprüche, wobei die Rekonstitutionszeit in Schritt d) kleiner als 10 min ist.

9. Zusammensetzung, umfassend lyophilisierten FVIII/VWF-Komplex, wobei die Konzentration von Arginin nach der Rekonstitution zwischen 42 und 98 mmol/l, die Konzentration von Histidin nach der Rekonstitution zwischen 10 und 24 mmol/l und die Konzentration von humanem Serumalbumin (HAS) nach der Rekonstitution zwischen 1,0 und 2,5 % (w/v) liegt, und wobei die FVIII-Potenz und die VWF-Potenz gleich oder größer als 200 IE/ml sind und eine FVIII-Dosis bis zu 3600 I.E./Fläschchen und eine VWF-Dosis bis zu 5040 I.E./Fläschchen.

10. Zusammensetzung nach Anspruch 9, wobei die Albuminkonzentration zwischen 1,2 und 2,3 % (w/v) liegt.

11. Zusammensetzung nach Anspruch 9 oder 10, wobei die Histidinkonzentration zwischen 13 und 21 mmol/l liegt.

12. Zusammensetzung nach einem der Ansprüche 9 bis 11, wobei die Argininkonzentration zwischen 50 und 90 mmol/l liegt.

13. Zusammensetzung nach einem der Ansprüche 9 bis 12, wobei die FVIII-Dosis zwischen 2400 und 3600 IE/Fläschchen und die VWF-Dosis zwischen 2160 und 5040 IE/Fläschchen liegt.

14. Zusammensetzung gemäß den Ansprüchen 9 bis 14, wobei die Rekonstitutionszeit der lyophilisierten Zusammensetzung weniger als 10 min beträgt.

15. Zusammensetzung nach einem der Ansprüche 9 bis 14 zur Verwendung bei der Behandlung von Hämophilie A und Morbus von Willebrand bei einem Probanden, der dies benötigt.

## Revendications

1. Procédé pour produire une composition pharmaceutique lyophilisée comprenant un complexe facteur VIII dérivé de plasma humain/facteur Von Willebrand (FVIIIIVWF), comprenant les étapes suivantes :
a) fournir une solution aqueuse initiale comprenant le *FVIII*/*VWF* avec au moins 90 UI de FVIII par mg de protéines totales, avec un rapport des activités du VWF/FVIII d'au moins 0,7,
b) ajouter de l'arginine, de l'histidine et de la sérum-albumine humaine (HSA),
c) lyophiliser la solution de *FVIII*/*VWF* obtenue à l'étape b), et
d) reconstituer le FVIII/VWF lyophilisé dans de l'eau pour injection, en obtenant ainsi un produit thérapeutique adapté au traitement de l'hémophilie A et de la maladie de Von Willebrand,
dans lequel, avant l'administration à un patient, l'arginine est à une concentration comprise entre 42 et 98 mmol/l, l'histidine est à une concentration comprise entre 10 et 24 mmol/l, l'albumine est à une concentration comprise entre 1,0 et 2,5 % (p/v), la puissance du FVIII et la puissance du VWF sont égales ou supérieures à 200 Ul/ml, et le produit obtenu a une dose de FVIII allant jusqu'à 3 600 Ul/flacon, et une dose de VWF allant jusqu'à 5 040 Ul/flacon.

2. Procédé selon la revendication 1, dans lequel la concentration d'albumine à l'étape d) est comprise entre 1,2 et 2,3 % (p/v).

3. Procédé selon la revendication 1 ou 2, dans lequel la concentration d'histidine à l'étape d) est comprise entre 13 et 21 mmol/l.

4. Procédé selon l'une quelconque des revendications précédentes, dans lequel la concentration d'arginine à l'étape d) est comprise entre 50 et 90 mmol/l.

5. Procédé selon l'une quelconque des revendications précédentes, comprenant en outre une étape de traitement thermique.

6. Procédé selon la revendication 5, dans lequel le complexe FVIII/VWF n'est pas affecté par ledit traitement thermique et est similaire au complexe FVIII/VWF présent dans le plasma.

7. Procédé selon l'une quelconque des revendications précédentes, dans lequel l'osmolalité du produit obtenu à l'étape d) est égale ou supérieure à 240 mOsm/kg.

8. Procédé selon l'une quelconque des revendications précédentes, dans lequel le temps de reconstitution à l'étape d) est inférieur à 10 min.

9. Composition comprenant un complexe *FVIII*/*VWF* lyophilisé, dans laquelle la concentration d'arginine après reconstitution est comprise entre 42 et 98 mmol/l, la concentration d'histidine après reconstitution est comprise entre 10 et 24 mmol/l, et la concentration de sérum-albumine humaine (HAS) après reconstitution est comprise entre 1,0 et 2,5 % (p/v), et dans laquelle la puissance du FVIII et la puissance du VWF sont égales ou supérieures à 200 Ul/ml, et une dose de FVII jusqu'à 3 600 Ul/flacon et une dose de VWF jusqu'à 5 040 Ul/flacon.

10. Composition selon la revendication 9, dans laquelle la concentration d'albumine est comprise entre 1,2 et 2,3 % (p/v).

11. Composition selon la revendication 9 ou 10, dans laquelle la concentration d'histidine est comprise entre 13 et 21 mmol/l.

12. Composition selon l'une quelconque des revendications 9 à 11, dans laquelle la concentration d'arginine est comprise entre 50 et 90 mmol/l.

13. Composition selon l'une quelconque des revendications 9 à 12, dans laquelle la dose de FVIII est comprise entre 2 400 et 3 600 Ul/flacon et la dose de VWF est comprise entre 2 160 et 5 040 Ul/flacon.

14. Composition selon l'une quelconque des revendications 9 à 14, dans laquelle le temps de reconstitution de la composition lyophilisée est inférieur à 10 min.

15. Composition selon l'une quelconque des revendications 9 à 14, pour une utilisation dans le traitement de l'hémophilie A et de la maladie de Von Willebrand chez un sujet qui en a besoin.
